# EUROPEAN PATENT SPECIFICATION

(11) **EP 4 475 763 B1**
(45) Date of publication and mention of the grant of the patent: **08.04.2026**
(21) Application number: 23718543.4
(22) Date of filing: 07.02.2023
(51) Int. Cl.: A61B 5/20, A61B 5/00, A61M 25/00

(54) **CATHETER DEVICE WITH SENSOR**
KATHETERVORRICHTUNG MIT SENSOR
DISPOSITIF DE CATHÉTER AVEC CAPTEUR

(30) Priority: 10.02.2022 US 202263308594 P
(43) Date of publication of application: 18.12.2024
(73) Proprietor: Hollister Incorporated, Libertyville, IL 60048 (US)
(72) Inventor: FARRELL, David, J., Libertyville, IL 60048 (US); PANESAR, Satwinder, S., Libertyville, IL 60048 (US); HORKAN, Carlos, Libertyville, IL 60048 (US); SILEIKA, Tadas, S., Libertyville, IL 60048 (US); DEAN, Nicole, L., Libertyville, IL 60048 (US); MURNAGHAN, Kevin, Libertyville, IL 60048 (US); STASIAK, Sandra, Libertyville, IL 60048 (US); KEYES, Niamh, Libertyville, IL 60048 (US)
(74) Representative: FRKelly
(86) International application number: PCT/US2023/062118
(87) International publication number: WO 2023/154700

(56) References cited:
- WO-A1-2018/058077
- CN-A- 112 304 930
- US-A1- 2016 346 504
- US-A1- 2019 366 039

## Description

The present application claims the benefit and priority to U.S. Provisional Patent Application No. 63/308,594, filed February 10, 2022.

### Field of the Disclosure

The present disclosure is directed to a urinary catheter product. More particularly, the present disclosure is directed to a urinary catheter product with an indicator. Even more particularly, the present disclosure is directed to a urinary catheter product with a biomarker strip within the lumen of the catheter configured to identify characteristics of a user's urine.

### Background

Intermittent catheterization is a good option for many users who suffer from various abnormalities of the urinary system. With the advent of intermittent urinary catheters, individuals with problems associated with the urinary system can conveniently self-catheterize to drain the individual's bladder. Individuals who suffer from urinary incontinence will self-catheterize several times a day.

Although catheters are typically prepared in sterile environments and are provided with safe handling instructions, catheter users are at risk of contracting a urinary tract infection due to several different factors. For example, the catheters may become contaminated during use and introduce bacteria into the urethra. The catheter also may carry bacteria along the urinary tract.

Urinary tract infections pose significant health risks to the user and are often not detected early enough before symptoms develop. Symptoms of urinary tract infections may include abdominal pain, back pain, pain with catheterization, and fever. One way to prevent urinary tract infections is by having a urodynamic evaluation done by a doctor. Such evaluations may be awkward for the individual and unrealistic for users that catheterize multiple times a day.

Therefore, there is a need for methods and devices that allow urinary catheter users to obtain information about the characteristics of the user's urine and alerting them as early as possible of a potential for a urinary tract infection. US2019366039 discloses systems and methods for inducing negative pressure in a portion of a urinary tract of a patient. WO2018/058077 discloses a system and method for urinary catheter bacterial detection.

### Summary

In one aspect, a urinary catheter product is provided. The urinary catheter product includes a catheter tube having a proximal insertion end and a distal drainage end. The urinary catheter product also includes a lumen extending from the proximal insertion end to the distal drainage end. The proximal insertion end has an opening for receiving urine in communication with the lumen and the distal drainage end has a drainage opening for draining urine from the lumen. Additionally, the urinary catheter product includes a biomarker strip within the lumen of the catheter product.

### Brief Description of the Drawings

Figure 1 is a top view of a urinary catheter product in accordance with the present disclosure;
Figure 2 is an enlarged partial top view of the biomarker strip of the urinary catheter product of Fig. 1;
Figure 3 is an enlarged partial top view of the catheter tube and distal drainage end of the urinary catheter product of Fig. 1;
Figure 3A is an enlarged partial top view of the catheter tube and distal drainage end of an embodiment of the urinary catheter product of Fig. 1;
Figure 4 is an enlarged partial side view of the catheter tube and distal drainage end of a first embodiment of the urinary catheter product; and
Figure 5 is an enlarged partial side view of the catheter tube and distal drainage end of a second embodiment of the urinary catheter product.

### Detailed Description of the Embodiments

A more detailed description of the device in accordance with the present disclosure is set forth below. It should be understood that the description of the specific devices below is intended to be exemplary, and not exhaustive of all possible variations or applications. Thus, the scope of the disclosure is not intended to be limiting and should be understood to encompass variations or embodiments that would occur to persons of ordinary skill.

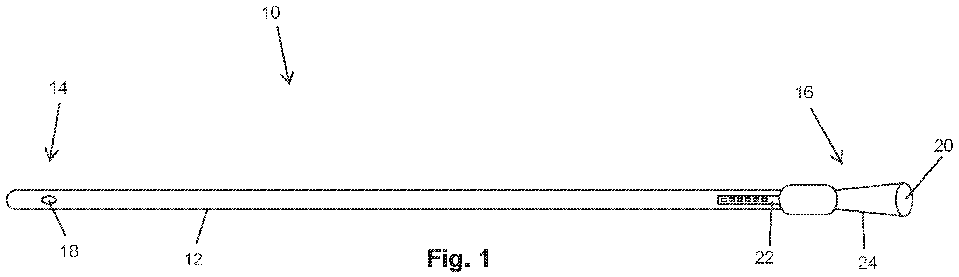

Fig. 1 shows a urinary catheter product 10. The catheter product 10 includes a catheter tube 12 having a proximal insertion end 14 and a distal drainage end 16. In one embodiment, the catheter tube 12 may be made of a transparent material, such as, but not limited to, PVC, TPE, TPO, TPU, and silicone. In another embodiment, the catheter tube 12 may be made of an opaque material, such as, but not limited to, a polymer containing an opaque filler, colorant, pigment, or coating such that the catheter is no longer transparent. Suitable polymers may be PVC, TPE, TPO, TPU, and silicone. A lumen extends from the proximal insertion end 14 to the distal drainage end 16. The proximal insertion end 14 includes one or more openings 18 for receiving urine in communication with the lumen. In one embodiment, the one or more openings 18 for receiving urine may be an eyelet. In another embodiment, the one or more openings 18 for receiving urine may be multiple eyelets. The one or more openings 18 for receiving urine may vary without departing from the scope of the present disclosure.

Furthermore, the distal drainage end 16 of the catheter tube 12 includes a drainage opening 20 for draining urine from the lumen. The distal drainage end 16 includes a drainage member 24, further defining the lumen of the catheter 10. The drainage member 24 may be, for example, but not limited to, a funnel or connector for connection to a drainage bag (not shown). Other drainage members known in the art may be used without departing from the scope of the present disclosure.

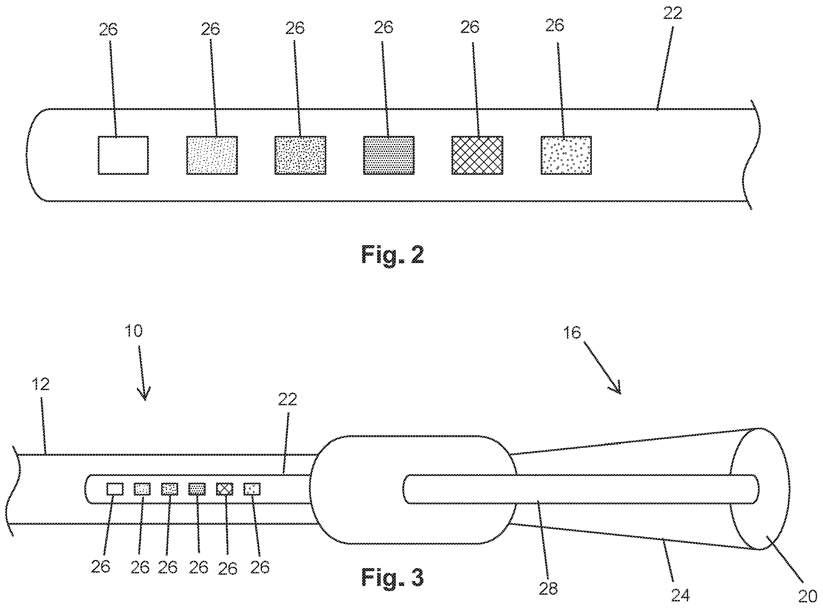

A biomarker strip 22 containing at least one indicator 26 may be inserted inside the lumen of the catheter product 10 near the distal drainage end 16 of the catheter product 10. The biomarker strip includes a front surface 23 and a rear surface 25. The biomarker strip 22 contains multiple indicators 26 (Fig. 2), preferably, on or associated with the front surface 23. The biomarker strip 22 is positioned in the lumen of the catheter product 10, preferably, without significantly impeding urine flow. In a preferred embodiment, the biomarker strip 22 is located immediately before the drainage member 24.

In one embodiment, the biomarker strip 22 is embedded in the distal drainage end 16 of the catheter product 10. The biomarker strip 22 may be secured to an inside surface of the catheter tube 12 inside the lumen of the catheter product 10 in a suitable manner. For example, but not limited to, the biomarker strip 22 may be secured to the inside surface of the catheter tube 12 by adhesives, mechanical fit, sonic welding, embedding, or other methods known to a person skilled in the art.

In another embodiment, the biomarker strip 22 may be embedded in, attached, or otherwise affixed to the drainage member 24. The biomarker strip 22 may be secured to the drainage member 24, for example, but not limited to, by adhesives, mechanical fit, sonic welding, embedding, or other methods known to a person skilled in the art.

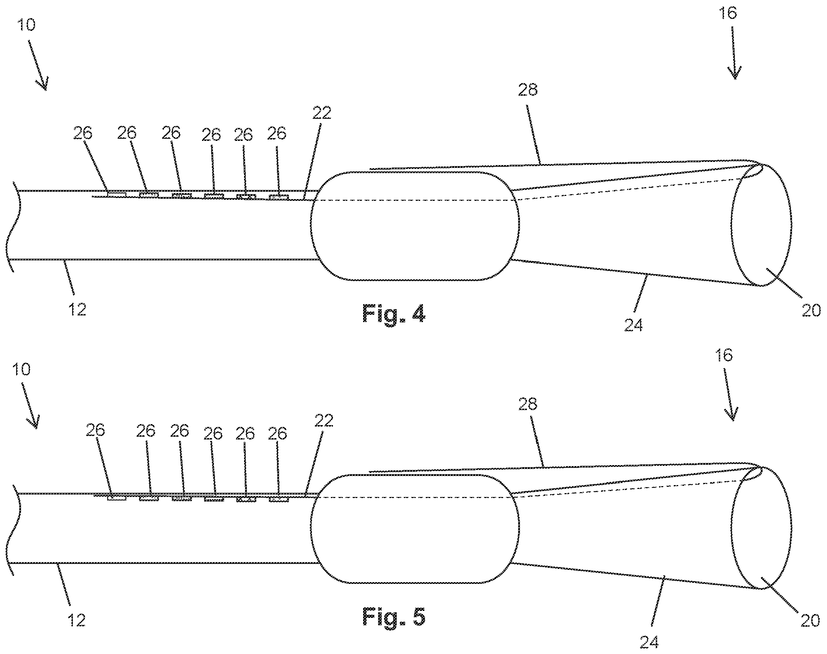

Turning to Figs. 4 and 5, these figures show the indicators 26 raised above the biomarker strip 22. It should be understood that the indicators 26 may be raised or significantly planar with the biomarker strip 22. In one embodiment, as shown in Fig. 4, the front surface 23 of the biomarker strip 22 and the indicators 26 may be positioned to abut an inner surface 27 of the catheter tube 12. The biomarker strip 22 may be secured to the catheter tube 12 with an adhesive placed in between the indicators 26, or by other methods known to a person skilled in the art. The adhesive adheres the front surface 23 of the biomarker strip 22 to the inner surface 27 of the catheter tube 12. In another embodiment, the indicators 26 may be positioned to face the lumen defined by the catheter tube 12. The biomarker strip 22 may be secured with an adhesive on the opposite side of the indicators 26, i.e., rear surface 25. Other methods of adhering the biomarker strip 22 to the catheter tube 12 known to a person skilled in the art may be used without departing from the scope of the disclosure.

The biomarker strip 22 contacts a user's urine during voiding of the bladder. When a user needs to void the bladder, the user will insert the proximal insertion end 14 of the catheter product 10 through the urethra until the opening 18 for receiving urine is in the bladder. To ease with insertion of the catheter, the catheter tube may include a hydrophilic coating on an outer surface of the catheter tube. The hydrophilic coating may be transparent. Once properly inserted, urine will then flow from the bladder, through the opening 18 on the proximal insertion end 14, through the lumen, contacting the biomarker strip 22, and ultimately out of the distal drainage end 16 of the catheter product 10. The urine contacts the indicators 26 directly or indirectly. For example, urine may flow directly over the indicators 26 or the biomarker strip 22 may absorb urine as it flows over the strip, wherein the urine absorbed into the strip contacts the indicators 26. For example, referring to Fig. 4, urine may contact the rear surface 25 of the biomarker strip 22 and be absorbed into the strip. The absorbed urine then comes into contact with the indicators 26.

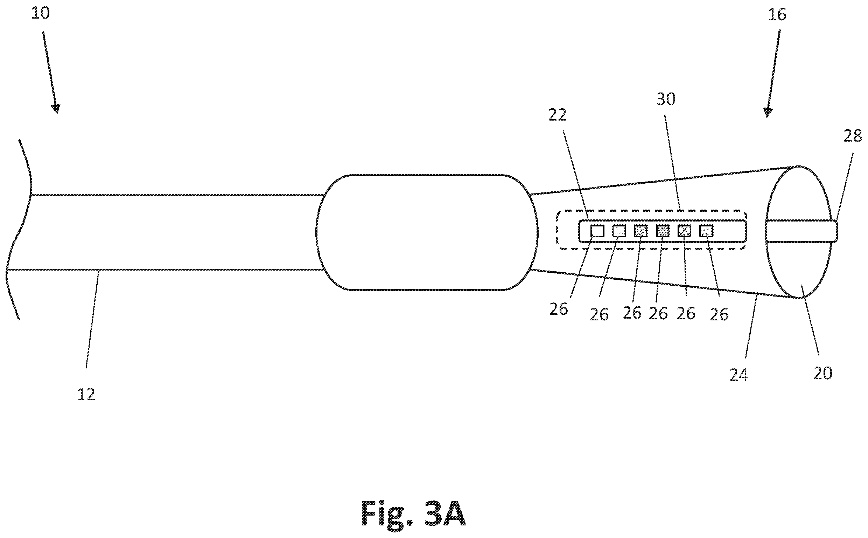

Fig. 3 shows an enlarged partial top view of the distal drainage end 16 of the catheter product 10, including the biomarker strip 22. The biomarker strip 22 includes a tab 28 extending out of the lumen of the catheter product 10. In one embodiment, the tab 28 may be folded over the drainage member 24 of the catheter product 10 and adhered to an outer surface of the drainage member 24 or catheter tube 12. In a preferred embodiment, the tab 28 may be folded over the funnel of the catheter product 10 and adhered to an outer surface of the funnel.

Fig. 3A shows an enlarged partial top view of another embodiment of the distal drainage end 16 of the catheter product 10, including the biomarker strip 22. The biomarker strip 22 may be embedded in, attached, or otherwise affixed to the drainage member 24. For example, the biomarker strip 22 may be affixed to the inner surface of the drainage member 24. The drainage member 24 may be, for example, a funnel. In one embodiment, the drainage member 24 may be made of an opaque material. A user may remove the biomarker strip 22 from the drainage member 24 using the tab 28 to view the biomarker strip 22. In another embodiment, the drainage member 24 is made of a transparent material and the biomarker strip 22 may be viewed through the drainage member 24. In yet another embodiment, the drainage member 24 may be made of an opaque material and has a transparent window 30. The biomarker strip 22 may be viewed by a user by looking through transparent window 30.

Fig. 2 shows an enlarged partial top view of the biomarker strip 22. The biomarker strip 22 contains at least one indicator 26 configured to identify characteristics of the urine. Preferably, the biomarker strip 22 includes more than one indicator 26 configured to identify characteristics of the urine. Each indicator 26 may test for one or more characteristics in the urine. The biomarker strip 22 may contain a protective coating which dissolves when it comes into contact with urine.

In one embodiment, the indicators 26 may be color-changing sensors. When the sensor contacts the urine, the sensor may change colors to indicate the presence of a particular characteristic. The sensors may be configured to detect, for example, but not limited to, urinary tract infections, urinary tract infection causative agents, the degree of urinary tract infection, monomicrobial or polymicrobial infection status, presence of biofilm, presence of antimicrobial resistant strains, proteases, ureases, pH, dissolved nitrogen, free nitrogen, leukocyte esterase, nitrite, lipoteichoic acid, enterotoxin, endotoxin, carcinomas, membrane-bound mucins, such as MUC1 and MUC 20, and the likes.

In an exemplary embodiment, when monitoring for a urinary tract infection and/or their causative agents, the colorimetric sensor may be configured to detect for, but not limited to, the presence of biofilm, the presence of antimicrobial resistant strains, proteases, ureases, pH, dissolved nitrogen, leukocyte esterase, nitrite, lipoteichoic acid, enterotoxin, and the likes.

In another exemplary embodiment, the color-changing sensors may be an enzyme-linked immunosorbent assay (ELISA).

In another exemplary embodiment, the color-changing sensors may be configured to monitor for damage to the urethral lining by detecting MUC1, MUC20, and other mucosal glycoproteins.

In yet another exemplary embodiment, the color-changing sensors may be configured to detect biomarkers that pertain to pathological conditions, including cancerous cell development.

In yet a further embodiment, the sensors may be configured to measure specific gravity, pH, leukocytes, hemoglobin, nitrites, ketones, bilirubin, urobilinogen, proteins, glucose.

After the user has voided the bladder, the user may view the biomarker strip 22 to assess the characteristics of the urine. In an embodiment where the catheter tube 12 is made of a transparent material, the user will be able to view the biomarker strip 22 through the catheter tube 12 without having to remove the biomarker strip 22. In an embodiment where the catheter tube 12 is opaque or has an opaque coating, the user may remove the biomarker strip 22 from the catheter tube 12 to view it. The biomarker strip 22 may be removed by peeling the tab 28 off the drainage member 24 and pulling the biomarker strip 22 out. The user may use a key card indicating what a color identifies to interpret any color change on the color-changing sensors. Optionally, the user may interpret the color change on the sensors using an electronic means. For instance, but not limited to, the results of the biomarker strip 22 may be interpreted using an app. In one embodiment, the user may use a smart device (such as a cellphone or tablet) to take a photo or scan of the biomarker strip 22. The smart device may include software, an application or connection via the internet to a software that analyzes the photo/scan and provides the analysis to the user or a healthcare provider. The analysis or other data may also be stored and readably retrievable for future viewing.

In another embodiment, the indicators 26 may be sensors with wireless communication capabilities. The sensors may be configured to detect, for example, but not limited to, urinary tract infections, urinary tract infection causative agents, the degree of urinary tract infection, monomicrobial or polymicrobial infection status, presence of biofilm, presence of antimicrobial resistant strains, proteases, ureases, pH, dissolved nitrogen, free nitrogen, leukocyte esterase, nitrite, lipoteichoic acid, enterotoxin, endotoxin, carcinomas, membrane-bound mucins, such as MUC1 and MUC 20, and the likes. The sensors may communicate the presence of a detected characteristic to a computing device with a display unit, for example, but not limited to, a user's smart phone. The information may be transmitted via RFID, NFC, and/or Bluetooth technology.

A baseline level of a desired biomarker can be established and subsequently monitored at predetermined intervals through the use of a software application on the computing device. As the detected level of the biomarker changes, feedback may be sent to the user or an associated clinician. The feedback may include, for example, but not limited to, notification of an imminent or ongoing infection, the type of infection present, or other abnormalities.

It will be understood that the embodiments and examples described above are illustrative of some of the applications of the principles of the present subject matter. Numerous modifications may be made by those skilled in the art without departing from the scope of the claimed subject matter, including those combinations of features that are individually disclosed or claimed herein. For these reasons, the scope hereof is not limited to the above description but is as set forth in the following claims.

## Claims

1. A urinary catheter product (10) comprising:
a catheter tube (12) having a proximal insertion end (14) and a distal drainage end (16);
a lumen extending from the proximal insertion end (14) to the distal drainage end (16);
the proximal insertion end (14) having an opening (18) for receiving urine in communication with the lumen;
the distal drainage end (16) having a drainage opening (20) for draining urine from the lumen;
the distal drainage end (16) including a drainage member (24) further defining the lumen;
a biomarker strip (22) within the lumen of the catheter product (10), **characterized in that** the biomarker strip (22) includes a tab (28) extending out of the lumen of the catheter product (10) and which is folded over the drainage member (24) and adhered to an outer surface of the drainage member (24); and
wherein the biomarker strip (22) is removable by peeling the tab (28) off the drainage member (24) and pulling the biomarker strip (22) out of the lumen.

2. The urinary catheter product (10) of claim 1, wherein the drainage member (24) is a funnel.

3. The urinary catheter product (10) of claim 1, wherein the drainage member (24) comprises a connector.

4. The urinary catheter product (10) of any one of claims 1-3, wherein the biomarker strip (22) is located near the distal drainage end (16) of the catheter tube (12).

5. The urinary catheter product (10) of any one of claims 1-4, wherein the biomarker strip (22) is affixed to an inside surface of the catheter tube (12).

6. The urinary catheter product (10) of any one of claims 1-5, wherein the biomarker strip (22) identifies characteristics of the urine.

7. The urinary catheter product (10) of any one of claims 1-6, wherein the biomarker strip (22) includes one or more indicators (26).

8. The urinary catheter product (10) of claim 7, wherein the biomarker strip (22) includes a front surface (23) and a rear surface (25), the one or more indicators (26) are located on the front surface (23) of the biomarker strip (22) and the rear surface (25) of the biomarker strip (22) being adhered to the inside surface of the catheter tube (12).

9. The urinary catheter product (10) of claim 7, wherein the biomarker strip (22) includes a front surface (23) and a rear surface (25), and the one or more indicators (26) are located on the front surface (23) of the biomarker strip (22) and the front surface (23) of the biomarker strip (22) being adhered to the inside surface of the catheter tube (12).

10. The urinary catheter product (10) of any one of claims 7-9, wherein the one or more indicators (26) of the biomarker strip (22) comprise color-changing sensors.

11. The urinary catheter product (10) of any one of claims 7-10, wherein the one or more indicators (26) have wireless communication capabilities.

12. The urinary catheter product (10) of claim 11, wherein the one or more indicators (26) communicate via RFID, NFC, and/or Bluetooth technology.

13. The urinary catheter product (10) of any one of claims 1-12, wherein the catheter tube (12) is transparent.

14. The urinary catheter product (10) of any one of claims 1-12, wherein the catheter tube (12) is opaque.

15. The urinary catheter product (10) of any of claims 1-14, further including a hydrophilic coating on an outer surface of the catheter tube (12), wherein the hydrophilic coating is transparent.

## Patentansprüche

1. Harnkatheterprodukt (10), umfassend:
einen Katheterschlauch (12), der ein proximales Einführungsende (14) und ein distales Drainageende (16) aufweist;
ein Lumen, das sich von dem proximalen Einführungsende (14) zu dem distalen Drainageende (16) erstreckt;
wobei das proximale Einführungsende (14) eine Öffnung (18) zum Aufnehmen von Urin in Verbindung mit dem Lumen aufweist;
wobei das distale Drainageende (16) eine Drainageöffnung (20) zum Entleeren von Urin aus dem Lumen aufweist;
wobei das distale Drainageende (16) ein Drainageelement (24) beinhaltet, welches das Lumen weiter definiert;
einen Biomarkerstreifen (22) innerhalb des Lumens des Katheterprodukts (10), **dadurch gekennzeichnet, dass** der Biomarkerstreifen (22) eine Lasche (28) beinhaltet, die sich aus dem Lumen des Katheterprodukts (10) heraus erstreckt und die über das Drainageelement (24) gefaltet ist und an einer Außenfläche des Drainageelements (24) haftet; und
wobei der Biomarkerstreifen (22) durch Abziehen der Lasche (28) von dem Drainageelement (24) und Herausziehen des Biomarkerstreifens (22) aus dem Lumen entfernbar ist.

2. Harnkatheterprodukt (10) nach Anspruch 1, wobei das Drainageelement (24) ein Trichter ist.

3. Harnkatheterprodukt (10) nach Anspruch 1, wobei das Drainageelement (24) einen Verbinder umfasst.

4. Harnkatheterprodukt (10) nach einem der Ansprüche 1 bis 3, wobei der Biomarkerstreifen (22) in der Nähe des distalen Drainageendes (16) des Katheterschlauchs (12) angeordnet ist.

5. Harnkatheterprodukt (10) nach einem der Ansprüche 1 bis 4, wobei der Biomarkerstreifen (22) an einer Innenfläche des Katheterschlauchs (12) befestigt ist.

6. Harnkatheterprodukt (10) nach einem der Ansprüche 1 bis 5, wobei der Biomarkerstreifen (22) Merkmale des Urins identifiziert.

7. Harnkatheterprodukt (10) nach einem der Ansprüche 1 bis 6, wobei der Biomarkerstreifen (22) einen oder mehrere Indikatoren (26) beinhaltet.

8. Harnkatheterprodukt (10) nach Anspruch 7, wobei der Biomarkerstreifen (22) eine Vorderfläche (23) und eine Rückfläche (25) beinhaltet, wobei der eine oder die mehreren Indikatoren (26) auf der Vorderfläche (23) des Biomarkerstreifens (22) angeordnet sind und die Rückfläche (25) des Biomarkerstreifens (22) an der Innenfläche des Katheterschlauchs (12) haftet.

9. Harnkatheterprodukt (10) nach Anspruch 7, wobei der Biomarkerstreifen (22) eine Vorderfläche (23) und eine Rückfläche (25) beinhaltet und der eine oder die mehreren Indikatoren (26) auf der Vorderfläche (23) des Biomarkerstreifens (22) angeordnet sind und die Vorderfläche (23) des Biomarkerstreifens (22) an der Innenfläche des Katheterschlauchs (12) haftet.

10. Harnkatheterprodukt (10) nach einem der Ansprüche 7 bis 9, wobei der eine oder die mehreren Indikatoren (26) des Biomarkerstreifens (22) farbveränderliche Sensoren umfassen.

11. Harnkatheterprodukt (10) nach einem der Ansprüche 7 bis 10, wobei der eine oder die mehreren Indikatoren (26) Fähigkeiten zur drahtlosen Kommunikation aufweisen.

12. Harnkatheterprodukt (10) nach Anspruch 11, wobei der eine oder die mehreren Indikatoren (26) über RFID-, NFC- und/oder Bluetooth-Technologie kommunizieren.

13. Harnkatheterprodukt (10) nach einem der Ansprüche 1 bis 12, wobei der Katheterschlauch (12) transparent ist.

14. Harnkatheterprodukt (10) nach einem der Ansprüche 1 bis 12, wobei der Katheterschlauch (12) undurchsichtig ist.

15. Harnkatheterprodukt (10) nach einem der Ansprüche 1 bis 14, ferner beinhaltend eine hydrophile Beschichtung auf einer Außenfläche des Katheterschlauchs (12), wobei die hydrophile Beschichtung transparent ist.

## Revendications

1. Produit de cathéter urinaire (10) comprenant :
un tube de cathéter (12) présentant une extrémité d'insertion proximale (14) et une extrémité de drainage distale (16) ;
une lumière s'étendant à partir de l'extrémité d'insertion proximale (14) vers l'extrémité de drainage distale (16) ;
l'extrémité d'insertion proximale (14) présentant un orifice (18) destiné à recevoir de l'urine, en communication avec la lumière ;
l'extrémité de drainage distale (16) présentant un orifice de drainage (20) destiné à évacuer de l'urine depuis la lumière ;
l'extrémité de drainage distale (16) comportant un élément de drainage (24) définissant en outre la lumière ;
une bandelette de biomarqueur (22) située dans la lumière du produit de cathéter (10), **caractérisée en ce que** la bandelette de biomarqueur (22) comporte une languette (28) s'étendant hors de la lumière du produit de cathéter (10), repliée sur l'élément de drainage (24) et collée sur une surface extérieure de l'élément de drainage (24) ; et
dans lequel la bandelette de biomarqueur (22) est amovible par décollement de la languette (28) de l'élément de drainage (24) et par extraction de la bandelette de biomarqueur (22) hors de la lumière.

2. Produit de cathéter urinaire (10) selon la revendication 1, dans lequel l'élément de drainage (24) est un entonnoir.

3. Produit de cathéter urinaire (10) selon la revendication 1, dans lequel l'élément de drainage (24) comprend un connecteur.

4. Produit de cathéter urinaire (10) selon l'une quelconque des revendications 1 à 3, dans lequel la bandelette de biomarqueur (22) est située près de l'extrémité de drainage distale (16) du tube de cathéter (12).

5. Produit de cathéter urinaire (10) selon l'une quelconque des revendications 1 à 4, dans lequel la bandelette de biomarqueur (22) est fixée à une surface intérieure du tube de cathéter (12).

6. Produit de cathéter urinaire (10) selon l'une quelconque des revendications 1 à 5, dans lequel la bandelette de biomarqueur (22) identifie des caractéristiques de l'urine.

7. Produit de cathéter urinaire (10) selon l'une quelconque des revendications 1 à 6, dans lequel la bandelette de biomarqueur (22) comporte un ou plusieurs indicateurs (26).

8. Produit de cathéter urinaire (10) selon la revendication 7, dans lequel la bandelette de biomarqueur (22) comporte une surface avant (23) et une surface arrière (25), les un ou plusieurs indicateurs (26) sont situés sur la surface avant (23) de la bandelette de biomarqueur (22) et la surface arrière (25) de la bandelette de biomarqueur (22) étant collée à la surface intérieure du tube de cathéter (12).

9. Produit de cathéter urinaire (10) selon la revendication 7, dans lequel la bandelette de biomarqueur (22) comporte une surface avant (23) et une surface arrière (25), et les un ou plusieurs indicateurs (26) sont situés sur la surface avant (23) de la bandelette de biomarqueur (22) et la surface avant (23) de la bandelette de biomarqueur (22) étant collée à la surface intérieure du tube de cathéter (12).

10. Produit de cathéter urinaire (10) selon l'une quelconque des revendications 7 à 9, dans lequel les un ou plusieurs indicateurs (26) de la bandelette de biomarqueur (22) comprennent des capteurs à changement de couleur.

11. Produit de cathéter urinaire (10) selon l'une quelconque des revendications 7 à 10, dans lequel les un ou plusieurs indicateurs (26) présentent des capacités de communication sans fil.

12. Produit de cathéter urinaire (10) selon la revendication 11, dans lequel les un ou plusieurs indicateurs (26) communiquent via la technologie RFID, NFC et/ou Bluetooth.

13. Produit de cathéter urinaire (10) selon l'une quelconque des revendications 1 à 12, dans lequel le tube du cathéter (12) est transparent.

14. Produit de cathéter urinaire (10) selon l'une quelconque des revendications 1 à 12, dans lequel le tube du cathéter (12) est opaque.

15. Produit de cathéter urinaire (10) selon l'une quelconque des revendications 1 à 14, comprenant en outre un revêtement hydrophile sur une surface extérieure du tube de cathéter (12), dans lequel le revêtement hydrophile est transparent.
